# EUROPEAN PATENT APPLICATION

(11) **EP 2 455 378 A1**
(43) Date of publication of application: **23.05.2012**
(21) Application number: 10014242.1
(22) Date of filing: 03.11.2010
(51) Int. Cl.: C07D 471/04, A61K 31/437, A61P 35/00

(54) **Carbazole and carboline derivatives, and preparation and therapeutic applications thereof**

(71) Applicant: Philip Morris Products S.A., 2000 Neuchâtel (CH)
(72) Inventor: The designation of the inventor has not yet been filed
(74) Representative: Maschio, Antonio

(57) **Abstract**

Compounds of general formula (I): 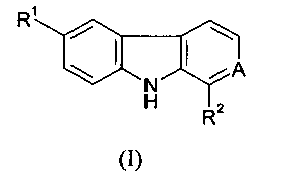 wherein A, R¹ and R² are defined herein are useful in the treatment or prevention of proliferative diseases including cancer.

## Description

### FIELD OF THE INVENTION

The present invention relates to carbazole and carboline derivatives, especially beta-carboline derivatives, compositions comprising them and their therapeutic uses. The present invention also relates to methods for preventing or treating various proliferative diseases and disorders by administering to a subject in need thereof one or more carbazole and carboline derivatives. In particular, the invention relates to methods for preventing or treating proliferative diseases such as cancer by administering to a subject in need thereof one or more carbazole and carboline derivatives. The present invention further relates to articles of manufacture and kits comprising one or more carbazole and carboline derivatives.

### BACKGROUND OF THE INVENTION

### Cancer and Proliferative Diseases

Cancer is one of the leading cause of death in the world. Currently, cancer therapy involves surgery, chemotherapy and/or radiation treatment but all of these approaches pose significant drawbacks for the patient. Surgery, for example, can be contraindicated due to the health of the patient or can be unacceptable to the patient. Additionally, surgery might not completely remove the neoplastic tissue. Radiation therapy is effective only when the irradiated neoplastic tissue exhibits a higher sensitivity to radiation than normal tissue, and radiation therapy often elicits serious side effects. Almost all chemotherapeutic agents are toxic, and chemotherapy can cause significant, and often dangerous, side effects, including severe nausea, bone marrow depression, immunosuppression, etc. Additionally, many tumor cells are resistant or develop resistance to chemotherapeutic agents through multi-drug resistance.

### Carbazole and Carboline Derivatives

Certain beta-carboline derivatives (pyrido[3,4-b]indoles) are already known to be of use as pharmaceutical compounds.

European Pat. No.0133000 discloses beta-carboline derivatives said to exhibit activity against DNA and RNA viruses and/or antibacterial activity. The substitution pattern of the disclosed beta-carboline derivatives is different from that of the compounds of the present invention as there is no aryl or heteroaryl at the 6-position and there is no alkyl substituent at the 1-position.

EP-A-0557497 and EP0110814 disclose beta-carboline derivatives said to be products such as tranquilizers, non-sedating anticonvulsants, anti-aggressives or anxiolytics with long lasting actions on the central nervous system.

EP-A-1209158 discloses beta-carboline derivatives said to be suitable for the production of pharmaceutical compounds for the prophylaxis or therapy disorders such as cancers in whose course an increased activity of IκB kinase is involved.

U.S. Pat. App. Pub. No. 2008/0069899 also relates to carboline derivatives which are of use in the treatment of cancer.

Therefore, there is a significant need for novel compounds and compositions, and methods that are useful for treating cancer or proliferative disease with minimal side effects, increased specificity and decreased toxicity.

### SUMMARY OF THE INVENTION

In a first aspect of the invention, there is provided a compound of general formula (I): 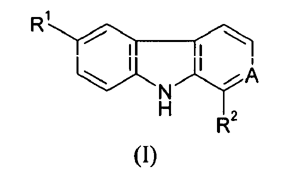 wherein
A is CH or N,
R¹ is aryl or heteroaryl selected from nitrogen containing compounds and oxygen containing compounds optionally substituted by up to four substituents independently selected from:
1. halo,
2. C₁-C₆ alkyl,
3. C₁-C₆ haloalkyl,
4. C₁-C₆ alkoxy,
5. C₁-C₆ haloalkoxy,
6. NHR³, (C₁-C₄ alkylene)NHR³;
7. NHC(O)R³, (C₁-C₄ alkylene)NHC(O)(C₁-C₆ alkyl),
8. C(O)NR³R⁴, (C₁-C₄ alkylene)C(O)N R³R⁴;
9. C(O)R³, (C₁-C₄ alkylene)C(O)R³;
10. C(O)O(C₁-C₆ alkyl), (C₁-C₄ alkylene)C(O)O(C₁-C₆ alkyl),
11. OCCO)(C₁-C₆ alkyl), (C₁-C₄ alkylene)OCCO)(C₁-C₆ alkyl),
12. OH, (C₁-C₄ alkylene)OH,
13. CN, (C₁-C₄ alkylene)CN,
14. aryl or heteroaryl selected from nitrogen containing compounds and oxygen containing compounds optionally substituted;
wherein each R³ and each R⁴ is independently H or C₁-C₆ alkyl;
R² is C₁-C₆ alkyl optionally substituted with halo;
or a pharmaceutically acceptable salt or solvate thereof;
provided that
if R¹ is a phenyl group, it is not substituted by any of the substituents 1 to 12 or 14 at the 4-position,
if R¹ is a phenyl group, it is not substituted by any of the substituents 2 to 14 at the 2-position
if R¹ is an aryl, it is not a naphthalenyl group, anthracenyl or phenanthrenyl group,
if R¹ is an heteroaryl selected from nitrogen containing compounds, it is not a pyrimidinyl or a pyridazinyl group.

It should be appreciated that certain compounds of the invention may contain one or more chiral atoms. Thus, the invention encompasses all stereoisomers, including enantiomers, diastereoisomers and mixtures thereof. In a preferred embodiment, the invention includes the racemic or either the R- or S-enantiomers of all the compounds described herein. The enantiomers may each be provided in a form substantially free of the other enantiomer (e.g., at least 75% free (w/w), at least 90% free (w/w) or at least 99% free (w/w)) or as mixtures (e.g., racemic mixtures). It should further be appreciated that the invention also extends to compounds in which one or more of the atoms has been replaced by an isotopic variant, for example one or more hydrogen atoms may be replaced by ²H or ³H and/or one or more carbon atoms may be replaced by ¹⁴C or ¹³C.

The compounds of general formula (I) are capable of interfering with tubulin polymerization inducing apoptosis and inhibiting cell metabolism and are therefore of use in medicine, especially in the treatment or prophylaxis of proliferative disorders and especially cancer.

In a second aspect of the invention, there is provided a use of the compound described above as a medicament and a use of the compound described above in medicine, especially in the treatment or prophylaxis of proliferative disorders and especially cancer.

In a third aspect of the invention, there is provided a use of the compound described above in the preparation or manufacture of an agent for the treatment or prevention of a proliferative disorder.

In a fourth aspect, there is provided a method for inhibiting tubulin polymerization by administering to a patient a therapeutically effective amount of the compound described above.

In a fith aspect, there is provided a method for the treatment or prevention of a proliferative disorder, the method providing administering to a patient in need of such treatment a therapeutically effective amount of the compound described above.

In a sixth aspect, there is provided a process for the preparation of the compound described above.

In a seventh aspect there is provided a pharmaceutical or veterinary composition comprising the compound described above together with a pharmaceutically or veterinarily acceptable carrier.

### DETAILED DESCRIPTION OF THE INVENTION

As used herein, the term "C₁-C₆ alkyl" refers to a straight or branched fully saturated hydrocarbon chain having from one to six carbon atoms. Examples include methyl, ethyl, n-propyl, iso-propyl, n-butyl, iso-butyl, tert-butyl, n-pentyl and n-hexyl.

The term "C₁-C₄ alkyl" refers to an alkyl group having from one to four carbon atoms. The term "C₁-C₂ alkyl" refers to an alkyl group having from one to two carbon atoms.

As used herein, the term "C₁-C₄ alkylene" refers to a C₁₋₄ bivalent group derived from a straight or branched-chain acyclic, cyclic, saturated, or unsaturated hydrocarbon by conceptual removal of two hydrogen atoms from different carbon atoms (i.e., -CH₂-, -CH₂CH₂-, -CH(CH₃)- , -CH₂CH₂CH₂-). References to "C₁-C₂ alkylene" refer to C₁₋₂ bivalent groups, i.e. -CH₂- or -CH₂CH₃-. References to "C₁ alkylene" refer to C₁ bivalent groups, i.e. -CH₂-.

The term "C₁-C₆ alkoxy" as used herein refers to a group O-C₁-C₆ alkyl. References to "C₁-C₄ alkoxy" refer to alkoxy groups having from 1 to 4 carbon atoms.

The term "halo" refers to fluoro, chloro, bromo or iodo.

As used herein, the terms "C₁-C₆ haloalkyl" and "C₁-C₆ haloalkoxy" refer to C₁-C₆ alkyl and C₁-C₆ alkoxy groups as defined above in which one or more hydrogen atoms have been replaced by a halo atom. Examples include trifluoromethyl, 2-chloroethyl, 3,3,3-trichloro-n-propyl and 1,1,2,2,2-pentafluoroethyl.

As used herein, the term "aryl" refers to a mono- or bi- or tricyclic group having from 5 to 14 carbon atoms and having aromatic character. The term also encompasses bicyclic or tricyclic groups in which an aromatic ring is fused to a partially or fully saturated ring.

The term "heteroaryl selected from nitrogen containing compounds and oxygen containing compounds" refers to an aryl group as defined above in which one or more of the carbon atoms are replaced by N or O. The term also encompasses bicyclic or tricyclic groups in which an aromatic ring is fused to a partially or fully saturated ring. Examples of such groups include pyrrolyl, triazolyl, tetrazolyl, imidazolyl, pyrazolyl, pyridyl, pyrazinyl, indolinyl, benzimidazolinyl, indolyl, benzimidazolyl, benzopyrazolyl, quinolinyl, isoquinolinyl, furanyl, benzofuranyl, coumarinyl, benzodioxolyl, dihyrodobenzofuranyl, dihydrobenzofuryl, dihydrobenzopyranyl, dihydroisobenzopyranyl, oxazolyl, dioxazolyl, isoxazolyl, chromenyl and chromanyl.

As used herein, the phrase "pharmaceutically acceptable salt" refers to pharmaceutically acceptable organic or inorganic salts of a compound of the invention. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counterions. Hence, a pharmaceutically acceptable salt can have one or more charged atoms and/or one or more counterion.

As used herein, the term "pharmaceutically acceptable solvate" refers to an association of one or more solvent molecules and a compound of the invention. Examples of solvents that form pharmaceutically acceptable solvates include, but are not limited to, water, isopropanol, ethanol, methanol, DMSO, ethyl acetate, acetic acid, and ethanolamine.

The present invention provides certain pyrido[3,4-b]indole compounds of general formula (I) as described above.

Particularly suitable compounds of general formula (I), are those in which R² is C₁-C₄ alkyl optionally substituted with halo, particularly methyl, ethyl or propyl optionally substituted with halo.

Particularly suitable compounds of general formula (I), are those in which A is nitrogen (N).

As set out above, R¹ is an aryl or heteroaryl selected from nitrogen containing compounds and oxygen containing compounds optionally substituted. Particularly suitable aryl and heteroaryl groups R¹ include phenyl, pyridyl, benzopyridyl, pyrrolyl, tetrazolyl, indolyl, indolinyl, furanyl, benzofuranyl, dihydrobenzofuranyl, and benzodioxolyl, optionally substituted as described above.

Still more suitable R¹ groups include phenyl, pyridyl, furanyl, benzofuranyl, indolyl and dihydrobenzofuranyl, optionally substituted as described above.

In particularly active compounds of the invention, R¹ is a phenyl group which is unsubstituted or which has a substituent at the 3-position or which has two substitutents at the 3- and the 5-position. Particularly suitable R¹ groups are phenyl groups which have a substituent at the 3-position or which have two substituents at the 3- and the 5-position.

Alternatively, R¹ may be pyridyl, 2,3-dihydroxybenzofuranyl, 5-substituted furanyl, benzo[d][1,3]dioxoyl and 1H-indol-5yl; in the case of the substituted furanyl groups, other substituents may also be present.

In particular, R¹ may be 3-pyridyl, 2,3-dihydroxybenzofuran-5-yl, 5-substituted furan-2-yl, preferably 5-methyl furan-2-yl, benzo[d][1,3]dioxo-5-yl and 1H-indol-5-yl, in the case of the substituted furanyl groups, other substituents may also be present.

The R¹ group may be substituted with one or more substituents as set out above. Usually, the R¹ group is unsubstituted or has 1 to 4 substituents, more commonly 1 to 3 substituents. In particularly suitable compounds of general formula (I), the R¹ group is unsubstituted or has 1 or 2 substituents.

Particularly suitable substituents for R¹ incude:
- halo,
- C₁-C₄ alkyl,
- C₁-C₄ haloalkyl,
- C₁-C₄ alkoxy,
- C₁-C₄ haloalkoxy,
- NHR⁵, (C₁-C₂ alkylene)NHR⁵;
- NHC(O)R⁵, (C₁-C₂ alkylene)NHC(O)(C₁-C₄ alkyl),
- C(O)NR⁵R⁶, (C₁-C₂ alkylene)C(O)NR⁵R⁶;
- C(O)R⁵; (C₁-C₂ alkylene)C(O)R⁵;
- C(O)O(C₁-C₄ alkyl), (C₁-C₂ alkylene)C(O)O(C₁-C₄ alkyl),
- OC(O)(C₁-C₄ alkyl), (C₁-C₂ alkylene)OC(O)(C₁-C₄ alkyl),
- OH, (C₁-C₂ alkylene)OH,
- CN, (C₁-C₂ alkylene)CN, or
- aryl or heteroaryl selected from nitrogen containing compounds and oxygen containing compounds optionally substituted;
wherein each R⁵ and each R⁶ is independently H or C₁-C₄ alkyl.

Any of the suitable substituents for R¹ described above may be substituted with one or more chloro or fluoro substitutents.

Still more suitable substituents include
- halo,
- C₁-C₂ alkyl, i.e. methyl or ethyl,
- C₁-C₂ alkoxy, i.e. methoxy or ethoxy
- NH₂ or CH₂NH₂,
- NHC(O)H, NHC(O)CH₃, NHC(O)CH₂CH₃, CH₂NHC(O)CH₃, CH₂NHC(O)CH₂CH₃, C(O)NH₂, C(O)NHCH₃, C(O)NHCH₂CH₃, CH₂C(O)NH₂, CH₂C(O)NHCH₃, CH₂C(O)NHCH₂CH₃, CH₂C(O)N(CH₃)₂, CH₂C(O)N(CH₂CH₃)₂,
- C(O)CH₃, C(O)CH₂CH₃, CHO, CH₂C(O)CH₃, CH₂C(O)CH₂CH₃, CH₂C(O)H,
- C(O)OCH₃, C(O)OCH₂CH₃, CH₂C(O)OCH₃, CH₂C(O)OCH₂CH₃,
- OC(O)CH₃, OC(O)CH₂CH₃, CH₂OC(O)CH₃, CH₂OC(O)CH₂CH₃,
- OH, CH₂OH, CH(CH₃)OH
- CN or CH₂CN, or
- aryl or heteroaryl selected from nitrogen containing compounds and oxygen containing compounds optionally substituted.

Any of the suitable substituents for R¹ described above may be substituted with one or more chloro or fluoro substituents.

Particularly suitable compounds of general formula (I) include:
1. 6-phenyl-1-methyl-9H-pyrido[3,4-b]indole;
2. 6-(3-chlorophenyl)-1-methyl-9H-pyrido[3,4-b]indole;
3. 6-(3,5-dichlorophenyl)-1-methyl-9H-pyrido[3,4-b]indole,
4. 6-(2-fluorophenyl)-1-methyl-9H-pyrido[3,4-b]indole;
5. 6-(3-fluorophenyl)-1-methyl-9H-pyrido[3,4-b]indole;
6. 6-(3-(trifluoromethyl)phenyl)-1-methyl-9H-pyrido[3,4-b]indole;
7. 6-(5-ethoxy-2-fluorophenyl)-1-methyl-9H-pyrido[3,4-b]indole;
8. 6-(3,5-dimethylphenyl)-1-methyl-9H-pyrido[3,4-b]indole;
9. 6-(3-methoxyphenyl)-1-methyl-9H-pyrido[3,4-b]indole;
10. 6-(3-(aminomethyl)phenyl)-1-methyl-9H-pyrido[3,4-b]indole;
11. 6-(3-(acetamidomethyl)phenyl)-1-methyl-9H-pyrido[3,4-b]indole;
12. 6-(3-aminocarbonylphenyl)-1-methyl-9H-pyrido[3,4-b]indole;
13. 6-(3-acetylphenyl)-1-methyl-9H-pyrido[3,4-b]indole;
14. 6-(3-methoxycarbonylphenyl)-1-methyl-9H-pyrido[3,4-b]indole;
15. 6-(3-(acetoxymethyl)phenyl)-1-methyl-9H-pyrido[3,4-b]indole;
16. 6-(3,5-bis(acetoxymethyl)phenyl)-1-methyl-9H-pyrido[3,4-b]indole;
17. 6-(3-hydroxyphenyl)-1-methyl-9H-pyrido[3,4-b]indole;
18. 6-(3-hydroxymethylphenyl)-1-methyl-9H-pyrido[3,4-b]indole;
19. 6-(3-(1-hydroxyethyl)phenyl)-1-methyl-9H-pyrido[3,4-b]indole;
20. 6-(3,5-bis(hydroxymethyl)phenyl)-1-methyl-9H-pyrido[3,4-b]indole;
21. 6-(3-cyanophenyl)-1-methyl-9H-pyrido[3,4-b]indole,
22. 6-(4-cyanophenyl)-1-methyl-9H-pyrido[3,4-b]indole;
23. 6-(3-cyanomethylphenyl)-1-methyl-9H-pyrido[3,4-b]indole;
24. 6-(5-cyano-2-fluorophenyl)-1-methyl-9H-pyrido[3,4-b]indole;
25. 6-(pyridine-3-yl)-1-methyl-9H-pyrido[3,4-b]indole;
26. 6-(1H-indol-5-yl)-1-methyl-9H-pyrido[3,4-b]indole;
27. 6-(((2H-tetrazol-5-yl)methyl)phenyl)-1-methyl-9H-pyrido[3,4-b]indole;
28. 6-(5-methylfuran-2-yl)-1-methyl-9H-pyrido[3,4-b]indole;
29. 6-(2,3-dihydrobenzofuran-5-yl)-1-methyl-9H-pyrido[3,4-b]indole;
30. 6-(benzo[d][1,3]dioxol-5-yl)-1-methyl-9H-pyrido[3,4-b]indole;
31. 6-(5-formylfuran-2-yl)-1-methyl-9H-pyrido[3,4-b]indole;
32. 6-(5-hydroxymethylfuran-2-yl)-1-methyl-9H-pyrido[3,4-b]indole;
33. 6-(3-aminophenyl)-1-methyl-9H-pyrido[3,4-b]indole;
34. 6-(3-formamidophenyl)-1-methyl-9H-pyrido[3,4-b]indole;
35. 6-(3-aminocarbonylphenyl)-1-ethyl-9H-pyrido[3,4-b]indole;
36. 6-(2,3-dihydrobenzofuran-5-yl)-1-trifluoromethyl-9H-pyrido[3,4-b]indole;
37. 6-(2,3-dihydrobenzofuran-5-yl)-1-ethyl-9H-pyrido[3,4-b]indole;
38. 6-(2,3-dihydrobenzofuran-5-yl)-1-propyl-9H-pyrido[3,4-b]indole;
39. 6-(2,3-dihydrobenzofuran-5-yl)-1-isopropyl-9H-pyrido[3,4-b]indole
and pharmaceutically acceptable salts or solvates thereof.

The compounds of general formula (I) wherein A is nitrogen, i.e. carboline derivatives may be prepared by the general method shown in scheme I.

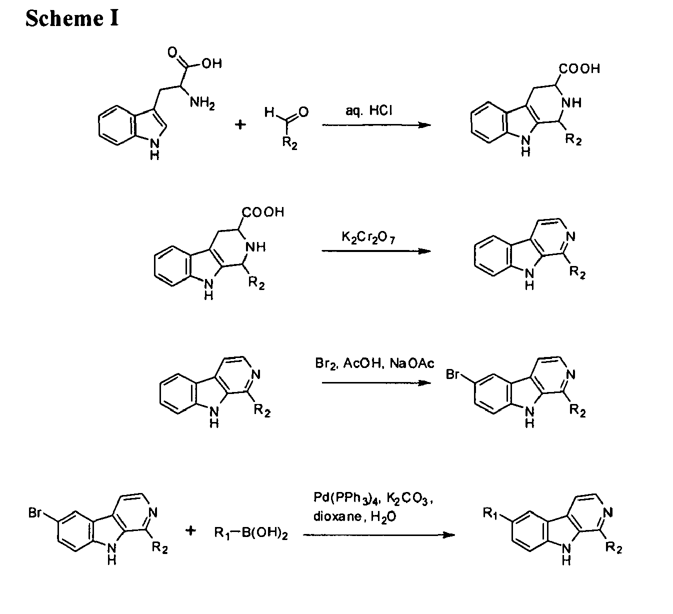

All of the starting materials shown in the scheme above are readily available or may be prepared by methods which are familiar to those of skill in the art.

Compounds of general formula (I) wherein A is nitrogen, may be prepared by a multi-step synthesis comprising a first step of adding an aldehyde comprising a R² substituents to tryptophane so as to form 1-R₂-2,3,4,9-tetrahydro-1H-pyrido[3,4-B]indole-3-carboxylic acid, a second step of oxidiation so as to form 1-R₂-9H-pyrido[3,4-b]indole and a third step of bromination of 1-R₂-9H-pyrido[3,4-b]indole so as to form 6-bromo-1-R₂-9H-pyrido[3,4-b]indole. Subsequently, any conventional Suzuki reaction can be done so as to modify the bromo substituent. Alternatively, should 6-bromo-1-R₂-9H-pyrido[3,4-b]indole be commercial, only the Suzuki reaction is required.

The compounds of general formula (I) wherein A is CH, i.e. carbazole compounds, may be prepared in methods similar to the one shown in scheme I, modifications may be done using conventional synthetic methodologies known to people having skills in the art.

Thus, in a further aspect of the invention there is provided a process for the preparation of a compound of general formula (I), the process comprising reacting a compound of the formula: 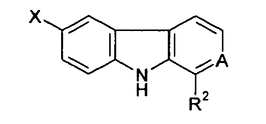 wherein A and R² are as defined for general formula (I) and X is a leaving group, especially halo and, in particular bromo; with a compound of the formula: R¹-B(OH)₂
where R¹ is as defined for general formula (I).

The reaction may be conducted under the conditions which are usual for a Suzuki reaction and such conditions are well known to those of skill in the art.

There is further provided a compound of general formula (I) as defined above as intermediate for the synthesis of a medicament.

In a further aspect, there is provided a compound of general formula (I) as defined above for use in medicine as intermediate for a medicament.

Therefore, in a further aspect, there is provided a compound of general formula (I) as defined above for use as medicament or for use in medicine, particularly in the treatment or prevention of a proliferative disorder, and especially for use as an inhibitor of tubulin polymerisation.

A variety of clinically compounds which demonstrate potent cytotoxicity and antitumor activity are known to effect their primary mode of action through an efficient inhibition of tubulin polymerization. Such compounds exhibit their anticancer properties by undergoing an initial interaction (binding) to the ubiquitous protein tubulin which in turn arrests the ability of tubulin to polymerize into microtubules which are essential components for cell maintenance and division. By disrupting the cellular microtubule structure, it results in mitotic arrest, as well as in the inhibition of the growth of epithelium of newly formed vasculature to shut down the blood supply to tumors (see Jordan et. al., (1998) Med. Res. Rev. 18: 259-296). As discussed above, the compounds of general formula (I) are capable of interfering with tubulin polymerization and inducing apoptosis and inhibiting cell metabolism. Without being bound by a particular theory, it appears that the compounds of general formula (I) may act by binding to an alpha- or beta-tubulin subunit in a cell and are therefore of use in the treatment or prophylaxis of proliferative disorders, which may be either non-cancerous or cancerous. For example, a compound of the invention may bind to an α- or β-tubulin subunit in a cancer or tumor cell and inhibit tubulin polymerization, thereby disrupting the cancer or tumor cell's ability to replicate. Alternatively, a compound of the invention may bind to an α- or β-tubulin subunit in endothelial cells of a vascularized tumor and cause a change in the shape of these cells. The change in shape of these endothelial cells results in constriction of blood vessels that supply a tumor with blood and oxygen, thereby cause the tumor to shrink or die.

The invention further provides the use of a compound of general formula (I) as a medicament, in the preparation of a medicament or for use in medicine, especially the use of a compound of general formula (I) in the preparation of an agent for the treatment or prevention of a proliferative disorder, especially in the preparation of an agent for inhibiting tubulin polymerization. The invention further provides the use of a compound of general formula (I) for the manufacture of a medicament, especially the manufacture of a medicament for the treatment or prevention of a proliferative disorder, especially the manufacture of a medicament for inhibiting tubulin polymerization. Also described herein are compounds of general formula (I) as defined above as tubulin polymerization inhibitors.

Furthermore, there is provided a method for the treatment or prevention of a proliferative disorder, the method providing administering to a patient in need of such treatment a therapeutically effective amount of a compound of general formula (I).

Furthermore, there is provided a method for inhibiting tubulin polymerization by administering to a patient a therapeutically effective amount of a compound of general formula (I).

As used herein, the term "therapeutically effective amount" refers to that amount of a therapy (e.g., a therapeutic agent) sufficient to result in the amelioration of one or more symptoms of a disorder, prevent advancement of a disorder, cause regression of a disorder, or to enhance or improve the therapeutic effect(s) of another therapy.

The patient may be a mammal, which may be a non-primate, for example a cow, pig, horse, cat, dog, rat or mouse but will more usually be a primate such as a monkey, for example a cynomolgous monkey, a chimpanzee or a human. Thus, the patient may be a farm animal (e.g., a horse, a cow, a pig, etc.) or a pet (e.g., a dog or a cat). Preferably, however, the patient is a human.

The proliferative disorder may be a non-cancerous disorder associated with cellular hyperproliferation, particularly of epithelial cells (e.g., as in asthma, COPD, pulmonary fibrosis, bronchial hyperresponsiveness, psoriasis, lymphoproliferative disorder, and seborrheic dermatitis), and endothelial cells (e.g., as in restenosis, hyperproliferative vascular disease, ocular neovascularisation, Behcet's Syndrome, arthritis, atherosclerosis, and macular degeneration). More usually, the non-cancerous proliferative disorder associated with cellular hyperproliferation is a condition such as Behcet's Syndrome, sarcoidosis, keloids, pulmonary fibrosis, and renal fibrosis.

The compounds of the invention are also of use for treating non-cancerous proliferative disorders in patients refractory to conventional therapies for such disorders.

In an alternative embodiment, the proliferative disorder is a cancer. Cancers that can be prevented, managed, treated or ameliorated in accordance with the methods of the invention include, but are not limited to, neoplasms, tumors (malignant and benign) and metastases, or any disease or disorder characterized by uncontrolled cell growth. The cancer may be a primary or metastatic cancer. Specific examples of cancers that can be prevented, managed, treated or ameliorated in accordance with the methods of the invention include, but are not limited to, cancer of the head, neck, eye, mouth, throat, esophagus, chest, bone, lung, colon, rectum, stomach, prostate, breast, ovaries, kidney, liver, pancreas, and brain. Additional cancers include, but are not limited to, the following: leukemias such as but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma, gallbladder cancers such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell cancer, adenocarcinoma, hypernephroma, fibrosarcoma, transitional cell cancer (renal pelvis and/or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America). It is also contemplated that cancers caused by aberrations in apoptosis can also be treated by the methods and compositions of the invention. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes.

In a specific embodiment, the cancer that is being prevented, managed, treated or ameliorated in accordance with the method of the invention is leukemia, colon cancer, pancreatic cancer, prostate cancer, breast cancer, bone cancer, melanoma, lung cancer or ovarian cancer.

In another embodiment, the cancer that is being prevented, managed, treated or ameliorated in accordance with the methods of the invention are metastatic tumors including, but not limited to, tumors that have or may metastasize to the bone (non-limiting examples are prostate, breast and lung cancers that have metastasized or have the potential to metastasize to the bone), tumors that have or may metastasize to the lung, tumors that have or may metastasize to the brain, or tumors that have or may metastasize to other organs or tissues of a subject.

The compounds of the invention may also be used in an *in vitro* or *ex vivo* method for the treatment of certain cancers, including, but not limited to leukemias and lymphomas, such treatment involving autologous stem cell transplants. This can involve a multi-step process in which the subject's autologous hematopoietic stem cells are harvested and purged of all cancer cells, the patient's remaining bone-marrow cell population is then eradicated *via* the administration of a high dose of a compound of the invention with or without accompanying high dose radiation therapy, and the stem cell graft is infused back into the subject. Supportive care is then provided while bone marrow function is restored and the subject recovers.

The compounds of the invention are of particular use where the patient is refractory or non-responsive to other treatments for cancer. The terms "non-responsive" and "refractory" describe patients treated with a currently available therapy (e.g., a prophylactic or therapeutic agent) for a proliferative disorder, which is not clinically adequate to relieve one or more symptoms associated with such disorder. Typically, such patients suffer from severe, persistently active disease and require additional therapy to ameliorate the symptoms associated with their disorder.

It is also possible to combine the compounds of the present invention with a radiation treatment. These treatments may be administered simultaneously, separately or sequentially. The treatment will be adapted by the practitioner as a function of the patient to be treated.

The compounds of general formula (I) may be used in combination with one or more additional therapeutic agents. The compound of general formula (I) and the other therapeutic agents may be administered in any order; thus, the one or more additional therapeutic agent may be administered prior to concomitantly with or subsequent to one another and the compounds of general formula (I).

Therefore, in a further aspect, the invention provides a product comprising a compound of general formula (I) and one or more of additional therapeutic agents as a combined preparation for simultaneous, separate or sequential use in the treatment of a proliferative disorder such as cancer.

The one or more additional therapeutic agents may be selected from any therapy (e.g., any prophylactic or therapeutic agent) which is known to be useful, has been used, or is currently being used for the prevention, treatment, management, or amelioration of one or more symptoms associated with a proliferative disorder.

Examples of such agents include, but are not limited to:
anti-inflammatory agents (e.g., corticosteroids (e.g., prednisone and hydrocortisone), glucocorticoids, steroids, non-steriodal anti-inflammatory drugs (e.g., aspirin, ibuprofen, diclofenac, and COX-2 inhibitors), beta-agonists, anticholinergic agents and methyl xanthines);
immunomodulatory agents;
gold injections;
sulphasalazine;
penicillamine;
anti-angiogenic agents (e.g., angiostatin, TNF-α antagonists (e.g., anti-TNFα antibodies), and endostatin);
anti-fibrotics;
antiemetic agents (e.g., metoclopromide, domperidone, prochlorperazine, promethazine, chlorpromazine, trimethobenzamide, ondansetron, granisetron, hydroxyzine, acethylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinols, thiethylperazine, thioproperazine and tropisetron);
opioids (e.g., morphine, heroin, hydromorphone, hydrocodone, oxymorphone, oxycodone, metopon, apomorphine, normorphine, etorphine, buprenorphine, meperidine, lopermide, anileridine, ethoheptazine, piminidine, betaprodine, diphenoxylate, fentanil, sufentanil, alfentanil, remifentanil, levorphanol, dextromethorphan, phenazocine, pentazocine, cyclazocine, methadone, isomethadone and propoxyphene), hematopoietic colony stimulating factors (e.g., filgrastim, pegfilgrastim sargramostim, molgramostim and epoetin alfa);
dapsone;
psoralens (e.g., methoxalen and trioxsalen);
antihistamines;
anti-malarial agents (e.g., hydroxychloroquine);
anti-viral agents; and
antibiotics (e.g., dactinomycin (formerly actinomycin), bleomycin, erythomycin, penicillin, mithramycin, and anthramycin (AMC)).

When the compounds of the invention are used in the treatment of cancer, the one or more additional agents may be an anti-cancer agent.

In certain embodiments, the anti-cancer agent is an immunomodulatory agent such as a chemotherapeutic agent. Alternatively, the anti-cancer agent may be an anti-angiogenic agent.

Examples of anti-cancer agents include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bisphosphonates (e.g., pamidronate (Aredria), sodium clondronate (Bonefos), zoledronic acid (Zometa), alendronate (Fosamax), etidronate, ibandornate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin-2 (including recombinant interleukin 2, or rIL2), interferon alpha-2a; interferon alpha-2b; interferon alpha-n1; interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; anti-CD2 antibodies; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (e.g., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, MA; U.S. Pat. No. 6,162,432); liarozole; linear polyamine analogue; lipophilic disaccharide peptide; lipophilic platinum compounds; lissoclinamide 7; iobapiatin; iombricine; iometrexoi; ionidamine; iosoxantrone; iovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; vorozole; zanoterone; zeniplatin; zilascorb; and zinostatin stimalamer.

The compounds of the invention will generally be provided in a pharmaceutical or veterinary composition. Therefore in a further aspect of the invention there is provided a pharmaceutical or veterinary composition comprising a compound of general formula (I) as defined above together with a pharmaceutically or veterinarily acceptable carrier.

The composition may be a single unit dosage form.

The composition of the invention may also include one or more additional therapeutic agents as defined above.

The carrier may comprise a diluent, adjuvant excipient and/or vehicle in which the compound of general formula (I) is administered. The choice of carrier will depend upon the chosen route of administration of the composition.

Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Examples of suitable pharmaceutical carriers are described in "Remington's Pharmaceutical Sciences" by E.W. Martin.

Typical pharmaceutical compositions and dosage forms comprise one or more excipients. Suitable excipients are well-known to those skilled in the art of pharmacy, and non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a patient and the specific active ingredients in the dosage form. The composition or single unit dosage form, if desired, can also contain minor amounts of wetting or emulsifying agents, or pH buffering agents.

Lactose-free compositions of the invention can comprise excipients that are well known in the art and are listed, for example, in the U.S. Pharmocopia (USP) SP (XXI)/NF (XVI). In general, lactose-free compositions comprise an active ingredient, a binder/filler, and a lubricant in pharmaceutically compatible and pharmaceutically acceptable amounts. Preferred lactose-free dosage forms comprise an active ingredient, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent preferably in purified form, together with a suitable amount of carrier so as to provide the form for proper administration to the patient. The formulation should suit the mode of administration. In a preferred embodiment, the pharmaceutical compositions or single unit dosage forms are sterile and in suitable form for administration to a subject, preferably an animal subject, more preferably a mammalian subject, and most preferably a human subject.

A pharmaceutical composition of the invention is formulated to be compatible with its intended route of administration. Examples of routes of administration include, but are not limited to, parenteral, e.g., intravenous, intradermal, subcutaneous, oral (e.g., inhalation), intranasal, transdermal (topical), transmucosal, intra-tumoral, intra-synovial and rectal administration. In a specific embodiment, the composition is formulated in accordance with routine procedures as a pharmaceutical composition adapted for intravenous, subcutaneous, intramuscular, oral, intranasal or topical administration to human beings. In a preferred embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocamne to ease pain at the site of the injection. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

The composition, shape, and type of dosage forms of the invention will typically vary depending on their use. For example, a dosage form used in the acute treatment of inflammation or a related disorder may contain larger amounts of one or more of the active ingredients it comprises than a dosage form used in the chronic treatment of the same disease. Also, the therapeutically effective dosage form may vary among different types of cancer. Similarly, a parenteral dosage form may contain smaller amounts of one or more of the active ingredients it comprises than an oral dosage form used to treat the same disease or disorder. These and other ways in which specific dosage forms encompassed by this invention will vary from one another will be readily apparent to those skilled in the art. See, e.g., Remington's Pharmaceutical Sciences, 18th ed., Mack Publishing, Easton PA (1990).

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration. Typical dosage forms of the invention comprise a compound of the invention, or a pharmaceutically acceptable salt, solvate or hydrate thereof lie within the range of from about 0.01 mg to about 10000 mg per day, given as a single once-a-day dose in the morning but preferably as divided doses throughout the day taken with food.

In still a further aspect of the invention, there is provided a kit comprising a compound or a composition of the invention in a suitable container. The kit may further comprise one or more additional pharmaceutical agents, either in the same or a separate container. If the additional pharmaceutical agent is in a separate container, it may be in a separate pharmaceutical composition.

The kit may further contain instructions for use of the compound or composition of the invention and, when present, the additional therapeutic agent.

The effective amount of the compound of the invention is an amount which is sufficient to reduce or ameliorate the severity, duration of a disorder or one or more symptoms thereof, prevent the advancement of a disorder, cause regression of a disorder, prevent the recurrence, development, or onset of one or more symptoms associated with a disorder, or enhance or improve the prophylactic or therapeutic effect(s) of another therapy.

When the compounds of the invention are used for the treatment of cancer, an effective amount refers to the amount of a therapy (e.g., a therapeutic agent) that inhibits or reduces the proliferation of cancerous cells, inhibits or reduces the spread of tumor cells (metastasis), inhibits or reduces the onset, development or progression of one or more symptoms associated with cancer, or reduces the size of a tumor. Preferably, a therapeutically effective of a therapy (e.g., a therapeutic agent) reduces the proliferation of cancerous cells or the size of a tumor by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99%, relative to a control or placebo such as phosphate buffered saline ("PBS").

The amount of the compound or composition of the invention which will be effective in the treatment, prevention, management or amelioration of a proliferative disorder will vary with the nature and severity of the disease or condition and the route by which the compound of the invention is administered. The frequency and dosage will also vary according to factors specific for each patient depending on the specific therapy (e.g. therapeutic or prophylactic therapy) administered, the severity of the disease or condition , the route of administration and the age, body weight, response and past medical history of the patient. Effective doses may be extrapolated from dose-response curves derived from *in vitro* or animal model test systems. Suitable regiments can be selected by one skilled in the art by considering such factors and by following, for example, dosages reported in the literature and recommended in the Physician's Desk Reference (57th ed., 2003).

In general, the recommended daily dose range of a compound of the invention for the conditions described herein lie within the range of from about 0.01 mg to about 10000 mg per day, given as a single once-a-day dose preferably as divided doses throughout a day. In one embodiment, the daily dose is administered twice daily in equally divided doses. Specifically, a daily dose range should be from about 5 mg to about 500 mg per day, more specifically, between about 10 mg and about 200 mg per day. In managing the patient, the therapy should be initiated at a lower dose, perhaps about 1 mg to about 25 mg, and increased if necessary up to about 200 mg to about 1000 mg per day as either a single dose or divided doses, depending on the patient's global response. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with individual patient response.

When the compounds of the invention are administered with one or more additional therapeutic agents as described above, the agents may be administered at intervals or cyclically (i.e. the compound of the invention and the one or more additional agents are administered in a sequence which is repeated one or more times). This type of cyclical therapy may be used in order to reduce the development of resistance to one or more of the agents and/or to avoid or reduce the side effects of one or more of the agent and/or to improve the efficacy of the treatment.

The pharmaceutical compositions and compounds of the invention can be tested in suitable animal model systems prior to use in humans. Such animal model systems include, but are not limited to, rats, mice, chicken, cows, monkeys, pigs, dogs, rabbits, etc. Any animal system well-known in the art may be used. In a specific embodiment of the invention, the pharmaceutical compositions and compounds of the invention are tested in a mouse model system. Such model systems are widely used and well-known to the skilled artisan. Pharmaceutical compositions or compounds of the invention can be administered repeatedly. Several aspects of the procedure may vary including, but not limited to, temporal regime for administration of the compositions or compounds.

The anti-cancer activity of the pharmaceutical compositions and compounds of the invention can be determined using any suitable animal model, including, but not limited to, SCID mice with a tumor or injected with malignant cells. Examples of animal models for lung cancer include, but are not limited to, lung cancer animal models described by Zhang & Roth (1994, In Vivo 8(5):755-69) and a transgenic mouse model with disrupted p53 function (see, e.g., Morris et al., 1998, J La State Med Soc 150(4):179-85). An example of an animal model for breast cancer includes, but is not limited to, a transgenic mouse that overexpresses cyclin D1 (see, e.g., Hosokawa et al., 2001, Transgenic Res 10(5):471-8). An example of an animal model for colon cancer includes, but is not limited to, a TCR b and p53 double knockout mouse (see, e.g., Kado et al., 2001, Cancer Res 61(6):2395-8). Examples of animal models for pancreatic cancer include, but are not limited to, a metastatic model of Panc02 murine pancreatic adenocarcinoma (see, e.g., Wang et al., 2001, Int J Pancreatol 29(1):37-46) and nu-nu mice generated in subcutaneous pancreatic tumors (see, e.g., Ghaneh et al., 2001, Gene Ther 8(3):199-208). Examples of animal models for non-Hodgkin's lymphoma include, but are not limited to, a severe combined immunodeficiency ("SCID") mouse (see, e.g., Bryant et al., 2000, Lab Invest 80(4):553-73) and an IgHmu-HOX11 transgenic mouse (see, e.g., Hough et al., 1998, Proc Natl Acad Sci USA 95(23):13853-8). An example of an animal model for esophageal cancer includes, but is not limited to, a mouse transgenic for the human papillomavirus type 16 E7 oncogene (see, e.g., Herber et al., 1996, J Virol 70(3):1873-81). Examples of animal models for colorectal carcinomas include, but are not limited to, Apc mouse models (see, e.g., Fodde & Smits, 2001, Trends Mol Med 7(8):369-73 and Kuraguchi et al., 2000, Oncogene 19(50):5755-63).

The effect of the pharmaceutical compositions and compounds of the invention on peripheral blood lymphocyte counts can be monitored/assessed using standard techniques known to one of skill in the art. Peripheral blood lymphocytes counts in a subject can be determined by, e.g., obtaining a sample of peripheral blood from said subject, separating the lymphocytes from other components of peripheral blood such as plasma using, e.g., Ficoll-Hypaque (Pharmacia) gradient centrifugation, and counting the lymphocytes using trypan blue. Peripheral blood T-cell counts in subject can be determined by, e.g., separating the lymphocytes from other components of peripheral blood such as plasma using, e.g., a use of Ficoll-Hypaque (Pharmacia) gradient centrifugation, labeling the T-cells with an antibody directed to a T-cell antigen such as CD3, CD4, and CD8 which is conjugated to FITC or phycoerythrin, and measuring the number of T-cells by FACS.

The toxicity and/or efficacy of the pharmaceutical compositions and compounds of the invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Pharmaceutical compositions and compounds of the invention that exhibit large therapeutic indices are preferred. While pharmaceutical compositions and compounds of the invention that exhibit toxic side effects may be used, care should be taken to design a delivery system that targets such compositions and compounds to the site of affected tissue in order to minimize potential damage to uninfected cells and, thereby, reduce side effects.

The data obtained from the cell culture assays and animal studies can be used in formulating a range of dosage of the pharmaceutical compositions and compounds of the invention for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography (HPLC) and radioimmunasssay (RIA). The pharmacokinetics of a prophylactic or therapeutic can be determined, e.g., by measuring parameters such as peak plasma level (Cₘₐₓ), area under the curve (AUC, which is measured by plotting plasma concentration of the agent versus time, and reflects bioavailability), half-life of the compound (t_{1/2}), and time at maximum concentration.

Efficacy in preventing or treating a proliferative disorder such as cancer may be demonstrated, e.g., by detecting the ability of the pharmaceutical compositions and compounds of the invention to reduce one or more symptoms of the proliferative disorder, to reduce the proliferation of cancerous cells, to reduce the spread of cancerous cells, or to reduce the size of a tumor. Efficacy in preventing or treating an inflammatory disorder may be demonstrated, e.g., by detecting the ability of the pharmaceutical compositions and compounds of the invention to reduce one or more symptoms of the inflammatory disorder, to decrease T cell activation, to decrease T cell proliferation, to modulate one or more cytokine profiles, to reduce cytokine production, to reduce inflammation of a joint, organ or tissue or to improve quality of life. Changes in inflammatory disease activity may be assessed through tender and swollen joint counts, patient and physician global scores for pain and disease activity, and the ESR/CRP. Progression of structural joint damage may be assessed by quantitative scoring of X-rays of hands, wrists, and feet (Sharp method). Changes in functional status in humans with inflammatory disorders may be evaluated using the Health Assessment Questionnaire (HAQ), and quality of life changes are assessed with the SF-36.

The invention is further described in the Examples below, which are provided to describe the invention in further detail. These examples, which set forth a preferred mode presently contemplated for carrying out the invention, are intended to illustrate and not to limit the invention.

### EXAMPLES

The invention will now be described in greater detail with reference to the following examples.

### Synthesis of Compounds

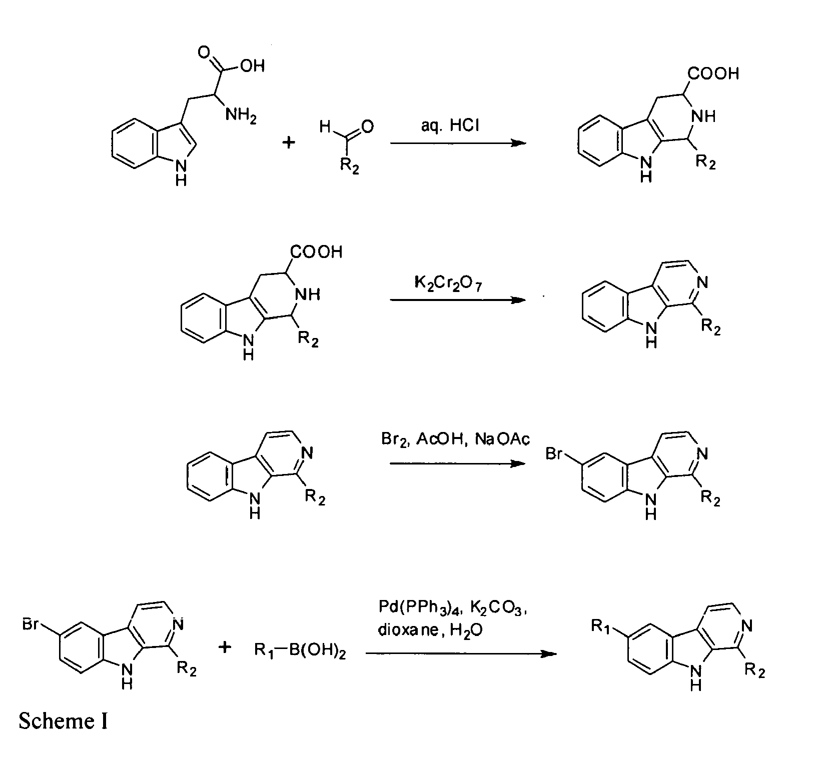

The compounds according to the invention are abbreviated as "E" in the Tables and comparative compounds are abbreviated as "C".

The structure of the compounds according to the present invention (E1-E39) and comparative ones (C1-C10) are illustrated in Table 1. The compounds according to the present invention (E1-E39) were made by an analogous method using appropriate starting materials as shown in scheme I.

**Table 1.**

| **No.** | **Structure** | **IC₅₀ (µM)** | **IC₅₀ (µM) caspase** |
|---|---|---|---|
| E1 | 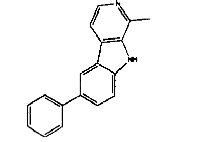 | 0.413 | |
| | 6-phenyl-1-methyl-9H-pyrido[3,4-b]indole | | |
| E2 | 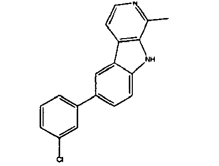 | 0.104 | 0.108 |
| | 6-(3-chlorophenyl)-1-methyl-9H-pyrido[3,4-b]indole | | |
| E3 | 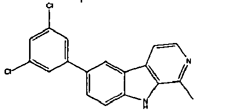 | 0.089 | 0.054 |
| | 6-(3,5-dichlorophenyl)-1-methyl-9H-pyrido[3,4-b]indole | | |
| E4 | 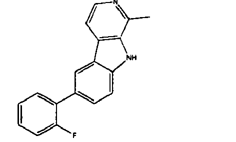 | 4.067 | |
| | 6-(2-fluorophenyl)-1-methyl-9H-pyrido[3,4-b]indole | | |
| E5 | 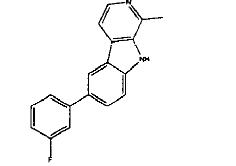 | 0.567 | |
| | 6-(3-fluorophenyl)-1-methyl-9H-pyrido[3,4-b]indole | | |
| E6 | 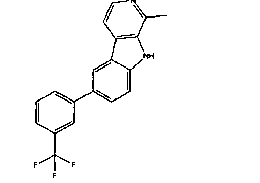 | 1.257 | |
| | 6-(3-(trifluoromethyl)phenyl)-1-methyl-9H-pyrido[3,4-b]indole | | |
| E7 | 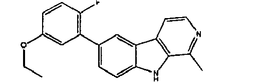 | 0.775 | |
| | 6-(5-ethoxy-2-fluorophenyl)-1-methyl-9H-pyrido[3,4-b]indole | | |
| E8 | 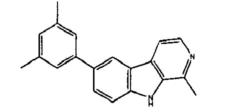 | 0.128 | |
| | 6-(3,5-dimethylphenyl)-1-methyl-9H-pyrido[3,4-b]indole | | |
| E9 | 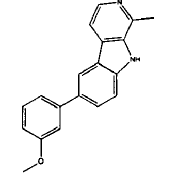 | 0.137 | 0.215 |
| | 6-(3-methoxyphenyl)-1-methyl-9H-pyrido[3,4-b]indole | | |
| E10 | 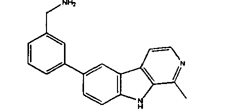 | 0.083 | |
| | 6-(3-(aminomethyl)phenyl)-1-methyl-9H-pyrido[3,4-b]indole | | |
| E11 | 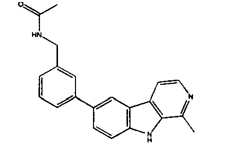 | 0.034 | |
| | 6-(3-(acetamidomethyl)phenyl)-1-methyl-9H-pyrido[3,4-b]indole | | |
| E12 | 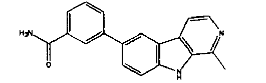 | 0.046 | |
| | 6-(3-aminocarbonylphenyl)-1-methyl-9H-pyrido[3,4-b]indole | | |
| E13 | 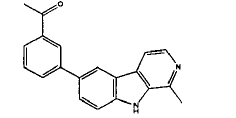 | 2.033 | |
| | 6-(3-acetylphenyl)-1-methyl-9H-pyrido[3,4-b]indole | | |
| E14 | 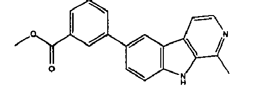 | 7.233 | |
| | 6-(3-methoxycarbonylphenyl)-1-methyl-9H-pyrido[3,4-b]indole | | |
| E15 | 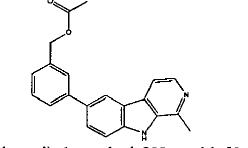 | 0.034 | |
| | 6-(3-(acetoxymethyl)phenyl)-1-methyl-9H-pyrido[3,4-b]indole | | |
| E16 | 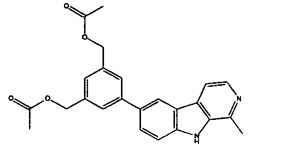 | 0.052 | |
| | 6-(3,5-bis(acetoxymethyl)phenyl)-1-methyl-9H-pyrido[3,4-b]indole | | |
| E17 | 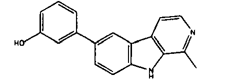 | 0.195 | |
| | 6-(3-hydroxyphenyl)-1-methyl-9H-pyrido[3,4-b]indole | | |
| E18 | 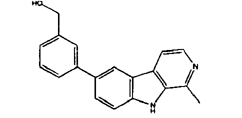 | 0.046 | |
| | 6-(3-hydroxymethylphenyl)-1-methyl-9H-pyrido[3,4-b]indole | | |
| E19 | 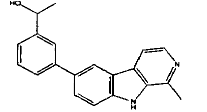 | 1.400 | |
| | 6-(3-(1-hydroxyethyl)phenyl)-1-methyl-9H-pyrido[3,4-b]indole | | |
| E20 | 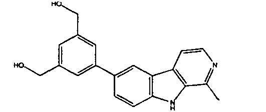 | 0.073 | |
| | 6-(3,5-bis(hydroxymethyl)phenyl)-1-methyl-9H-pyrido[3,4-b]indole | | |
| E21 | 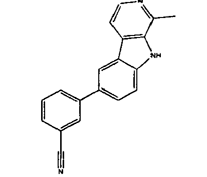 | 0.050 | 0.061 |
| | 6-(3-cyanophenyl)-1-methyl-9H-pyrido[3,4-b]-indole | | |
| E22 | 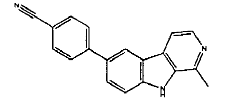 | 5.000 | |
| | 6-(4-cyanophenyl)-1-methyl-9H-pyrido[3,4-b]indole | | |
| E23 | 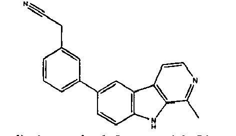 | 0.064 | |
| | 6-(3-cyanomethylphenyl)-1-methyl-9H-pyrido[3,4-b]indole | | |
| E24 | 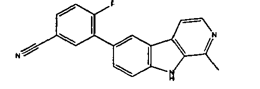 | 0.968 | |
| | 6-(5-cyano-2-fluoropheny)-1-metnyl-9H-pyriodo[3,4-b]indole | | |
| E25 | 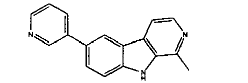 | 0.602 | |
| | 6-(pyridine-3-yl)-1-methyl-9H-pyrido[3,4-b]indole | | |
| E26 | 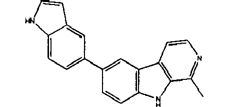 | 0.374 | |
| | 6-(1H-indol-5-yl)-1-methyl-9H-pyrido[3,4-b]indole | | |
| E27 | 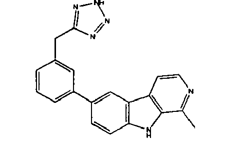 | 2.200 | |
| | 6-(((2H-tetrazol-5-yl)methyl)phenyl)-1-methyl-9H-pyrido[3,4-b]indole | | |
| E28 | 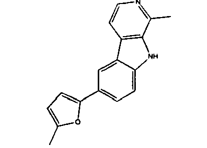 | 0.130 | |
| | 6-(5-methylfuran-2-yl)-1-methy-9H-pyrido[3,4-b]indole | | |
| E29 | 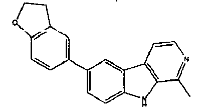 | 0.064 | 0.128 |
| | 6-(2,3-dihydrobenzofuran-5-yl)-1-methyl-9H-pyrido[3,4-b]indole | | |
| E30 | 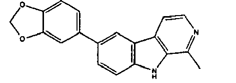 | 0.490 | |
| | 6-(benzo[d][1,3]dioxol-5-yl)-1-methyl-9H-pyrido[3,4-b]indole | | |
| E31 | 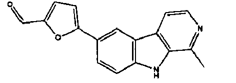 | 2.287 | |
| | 6-(5-formylfuran-2-yl)-1-methyl-9H-pyrido[3,4-b]indole | | |
| E32 | 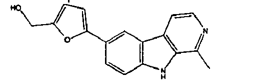 | 3.660 | |
| | 6-(5-hydroxymethylfuran-2-yl)-1-methyl-9H-pyrido[3,4-b]indole | | |
| E33 | 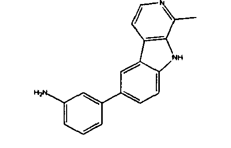 | 0.0960 | |
| | 6-(3-aminophenyl)-1-methyl-9H-pyrido[3,4-b]indole | | |
| E34 | 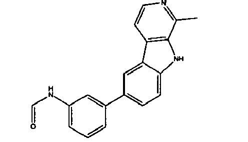 | 0.028 | |
| | 6-(3-formamidophenyl)-1-methyl-9H-pyridol[3,4-b]indole | | |
| E35 | 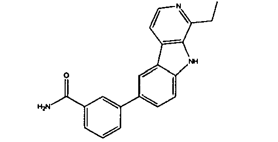 | 0.004 | |
| | 6-(3-aminocarbonylphenyl)-1-ethyl-9H-pyrido[3,4-b]indole | | |
| E36 | 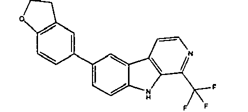 | 0.096 | |
| | 6-(2,3-dihydrobenzofuran-5-yl)-1-trifluoromethyl-9H-pyrido[3,4-b]indole | | |
| E37 | 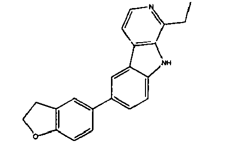 | 0.004 | |
| | 6-(2,3-dihydrobenzofuran-5-yl)-1-ethyl-9H-pyrido[3,4-b]indole | | |
| E38 | 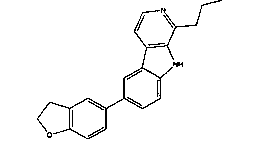 | 0.230 | |
| | 6-(2,3-dihydrobenzofuran-5-yl)-1-propyl-9H-pyrido[3,4-b]indole | | |
| E39 | 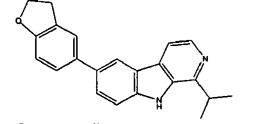 | 0.302 | |
| | 6-(2,3-dihydrobenzofuran-5-yl)-1-isopropyl-9H-pyrido[3,4-b]indole | | |
| C1 | 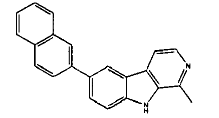 | not active | |
| | 6-(naphthalen-2-yl)-1-methyl-9H-pyrido[3,4-b]indole | | |
| C2 | 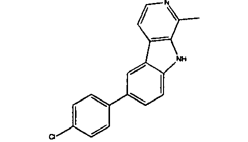 | not active | |
| | 6-(4-chlorophenyl)-1-methyl-9H-pyrido[3,4-b]indole | | |
| C3 | 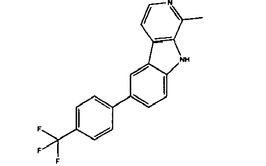 | not active | |
| | 6-(4-(trifluoromethyl)phenyl)-1-methyl-9H-pyrido[3,4-b]indole | | |
| C4 | 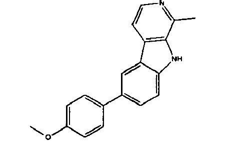 | not active | |
| | 6-(4-methoxyphenyl)-1-methyl-9H-pyrido[3,4-b]indole | | |
| C5 | 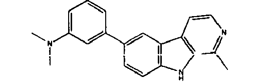 | 11.533 | |
| | 6-(3-(N,N-dimethylamino)phenyl)-1-methyl-9H-pyrido[3,4-b]indole | | |
| C6 | 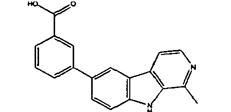 | not active | |
| | 6-(3-carboxyphenyl)-1-methyl-9H-pyrido[3,4-b]indole | | |
| C7 | 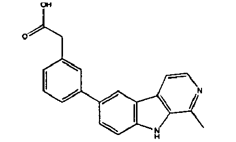 | not active | |
| | 6-(3-carboxymethylphenyl)-1-methyl-9H-pyrido[3,4-b]indole | | |
| C8 | 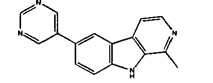 | 27.750 | |
| | 6-(pyrimidin-5-yl)-1-methyl-9H-pyrido[3,4-b]indole | | |
| C9 457 | 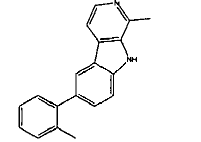 | 41.787 | |
| | 6-(2-methylphenyl)-1-methyl-9H-pyrido[3,4-b]indole | | |
| C10 | 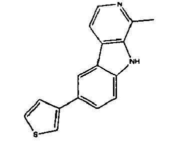 | not active | |
| | 6-(thiophen-3-yl)-1-methyl-9H-pyrido[3,4-b]indole | | |

As can be appreciated by chemists possessing ordinary skill in the art, the synthetic scheme described above is for illustrative purposes only and may be modified using conventional synthetic methodology to produce any compound of general formula (I). Depending on precisely how the synthetic schemes are modified, the specific reaction conditions might also require modification. Such modifications may, for instance, involve the use of higher or lower temperature or pressure conditions than those reported herein or the addition of further synthetic steps, such as functional group transformations. However, since progress of the reactions is easily monitored by techniques such as high performance liquid chromatography, gas chromatography, mass spectroscopy, thin layer chromatography, nuclear magnetic resonance spectroscopy and the like, such modifications are well within the skill of the art. The purity and identity of all synthesized compounds E1-E30 was confirmed by HPLC-MS analysis.

### Intermediate I1: 6-bromo-1-methyl-9H-pyrido[3,4-b]indole

To a solution of 1-methyl-9H-pyrido[3,4-b]indole (harmane; 2 g, 11 mmol) and sodium acetate (1.81 g 22.1 mmol in acetic acid (22 mL) a solution of bromine (1.76 g, 11 mmol) acetic acid (2 ml) was added. After stirring at room temperature for 1 h 40 min the reaction mixture was poured into 200 mL 2M aq. NaOH. The solution was adjusted with NaOH to a pH of 12 and the resulting precipitate was filtered and dried to give 2.9 g of 6-bromo-1-methyl-9H-pyrido[3,4-b]indole (I1).

### Intermediate 12: 6-bromo-1-ethyl-9H-pyrido[3,4-b]indole

L-tryptophane (8.0 g; 39 mmol) was dissolved in 0.12 M aq. HCl (590 mL) and propanal (4.64 g; 80 mmol) was added. After stirring the reaction mixture at room temperature for 17 h, acetic acid (20 mL) and potassium dichromate (9.25 g) were added and the solution heated to 80°C for 3.5 h. Excessive dichromate was reduced by addition of sodium sulfite (9.4 g) and the pH adjusted to ~12 with 10% aq. sodium hydroxide. The precipitate was filtered, washed with water, and dried to yield 6.3 g of 1-ethyl-9H-pyrido[3,4-b]indole. Bromination using a procedure analogous to the one described for I1 gave 6-bromo-1-ethyl-9H-pyrido[3,4-b]indole (I2).

### Intermediate I3: 6-bromo-1-trifluoromethyl-9H-pyrido[3,4-b]indole

L-tryptophane (4.0 g; 20 mmol) was dissolved in 0.1 M aq. HCl (300 mL) and trifluoroacetaldehyde (3.5 mL; 40 mmol) was added. After stirring the reaction mixture at room temperature for 17 h, acetic acid (10 mL) and potassium dichromate (4.5 g) were added and the solution heated to 80°C for 4 h. Excessive dichromate was reduced by addition of sodium sulfite and the pH was adjusted to ~12 with 10% aq. sodium hydroxide. The solution was extracted twice with dichloromethane (300 mL), and the combined organic phases dried to yield 2.5 g of 1-trifluoromethyl-9H-pyrido[3,4-b]indole. Bromination using a procedure analogous to the one described for 11 gave 6-bromo-1-trifluoromethyl-9H-pyrido[3,4-b]indole (I3).

### Intermediate I4: 6-bromo-1-propyl-9H-pyrido[3,4-b]indole

L-tryptophane (4.0 g; 20 mmol) was dissolved in 0.1 M aq. HCl (300 mL) and butyraldehyde (2.88 g; 40 mmol) was added. After stirring the reaction mixture at room temperature for three days the white precipitate was filtered, washed and dried to give 3.41 g of 1-propyl-1,2,3,4-tetrahydro-β-carboline-3-carboxylic acid. An aliquot of this compound (1.5 g) was dissolved in 1M HCl (100 mL) and glacial acetic acid (5 mL) and potassium dichromate (2g in 20 mL water) were added. After heating to 80°C for 20 minutes the reaction mixture was cooled and sodium sulfite (2g in 20 mL water) were added. With conc. NaOH the solution was adjusted to pH ~12 and then extracted twice with dichloromethane. Drying the combined dichloromethane phases yielded 1-propyl-9H-pyrido[3,4-b]indole (1.2 g), which was brominated to give 6-bromo-1-propyl-9H-pyrido[3,4-b]indole (I4) using the procedure described in I1.

### Intermediate I5: 6-bromo-1-isopropyl-9H-pyrido[3,4-b]indole

L-tryptophane (4.0 g; 20 mmol) was dissolved in 0.1 M aq. HCl (300 mL) and butyraldehyde (3.7 mL; 40 mmol) was added. After heating the reaction mixture at 104°C for 24h, potassium dichromate (7 g) was added and the solution heated to 64 °C for 30 minutes. Excessive dichromate was reduced by addition of sodium sulfite, and the pH was adjusted to ~7 with 10 % aq. sodium hydroxide. The precipitate was filtered, washed with water, and dried to yield 1.1 g of 1-isopropyl-9H-pyrido[3,4-b]indole. Bromination using a procedure analogous to the one described for I1 gave 6-bromo-1-isopropyl-9H-pyrido[3,4-b]indole (I5).

### Example E1: 6-phenyl-1-methyl-9H-pyrido[3,4-b]indole

Phenylboronic acid (0.459 mmol; 56 mg), 6-bromo-1-methyl-9H-pyrido[3,4-b]indole (0.383 mmol; 100 mg), K₂CO₃ (0.766 mmol; 106 mg) and tetrakis(triphenylphosphine)palladium (0.00191 mmol; 22 mg) were placed into vial. A degassed solvent mixture of dioxane:water (3:1; 2 mL) was added. After heating the sealed vial (10 min; 150°C), the reaction mixture was filtered through celite and evaporated to dryness. The crude product was purified first by flash chromatography on silica eluting with n-hexane/acetone and then by adsorption onto a strong cation exchanger cartridge, which was eluted with methanol/aq. ammonia (20%). A final purification step using preparative reversed-phase HPLC with a gradient from aqueous 0.1% ammonia/acetonitrile (50:50) to aqueous 0.1% ammonia/acetonitrile (5:95) gave 12 mg of E1.

### Example E2: 6-(3-chlorophenyl)-1-methyl-9H-pyrido[3,4-b]indole

3-chlorophenylboronic acid (0.459 mmol; 71.8 mg), 6-bromo-1-methyl-9H-pyrido[3,4-b]indole (0.383 mmol; 100 mg), K₂CO₃ (0.766 mmol; 106 mg) and tetrakis(triphenylphosphine)palladium (0.00191 mmol; 22 mg) were placed into vial. A degassed solvent mixture of dioxane:water (3:1; 2 mL) was added. After heating the sealed vial (10 min; 120°C) the reaction mixture was filtered through celite and and the filtrate adsorbed onto a cation exchanger cartridge (SCX), which was then washed with methanol and eluted with methanol/aq. ammonia (20%). A final purification step using preparative reversed-phase HPLC with a gradient from aqueous 0.1% ammonia/acetonitrile (50:50) to aqueous 0.1% ammonia/acetonitrile (5:95) gave 23.6 mg of E2.

### Example E3: 6-(3,5-dichlorophenyl)-1-methyl-9H-pyrido[3,4-b]indole

E3 (3 mg) was prepared from 3,5-dichlorophenylboronic acid using a modification of the procedure described in example E25. After the first heating step, the reaction mixture was heated for an additional period of 60 hours at 95°C.

### Example E4: 6-(2-fluorophenyl)-1-methyl-9H-pyrido[3,4-b]indole

E4 (25 mg) was prepared from 2-fluorophenylboronic acid using the procedure described in example E1.

### Example E5: 6-(3-fluorophenyl)-1-methyl-9H-pyrido[3,4-b]indole

E5 (32 mg) was prepared from 3-fluorophenylboronic acid using the procedure described in example E2.

### Example E6: 6-(3-(trifluoromethyl)phenyl)-1-methyl-9H-pyrido[3,4-b]indole

E6 (10 mg) was prepared from 3-(trifluoromethyl)phenylboronic acid using the procedure described in example E2.

### Example E7: 6-(5-ethoxy-2-fluorophenyl)-1-methyl-9H-pyrido[3,4-b]indole

E7 (28 mg) was prepared from 5-ethoxy-2-fluorophenylboronic acid using a modification of the procedure described in example E25. After the first heating step, the reaction mixture was heated for an additional period of 12 hours at 95°C.

### Example E8: 6-(3,5-dimethylphenyl)-1-methyl-9H-pyrido[3,4-b]indole

The synthesis and purification of E8 was carried out applying the procedure described for example E21 using 3,5-dimethylphenylboronic acid. The reaction yielded 71 mg of E8.

### Example E9: 6-(3-methoxyphenyl)-1-methyl-9H-pyrido[3,4-b]indole

E9 (28 mg) was prepared from 3-methoxyphenylboronic acid using the procedure described in example E2.

### Example E10: 6-(3-(aminomethyl)phenyl)-1-methyl-9H-pyrido[3,4-b]indole

6-(3-cyanophenyl)-1-methyl-9H-pyrido[3,4-b]indole (example E21; 10 mg; 0.035 mmol) was dissolved in methanol (2 mL). Solutions of sodium borohydride and CoCl₂ x 7H₂O (each 20 mg/mL methanol) were added dropwise while monitoring the reaction by LC-MS. After completion of the reaction, water (8 mL) was added and the pH was adjusted to pH ~9 with concentrated HCl. The product, E10(8.2 mg), was purified over an C18 cartridge.

### Example E11: 6-(3-(acetamidomethyl)phenyl)-1-methyl-9H-pyrido[3,4-b]indole

6-(3-aminomethylphenyl)-1-methyl-9H-pyrido[3,4-b]indole (example E10; 6.7 mg; 0.023 mmol) was dissolved in pyridine (0.1 mL) and acetic anhydride (0.1 ml). After one hour at 40°C, the reaction was stopped by adding water (5 mL) to the reaction mixture. Purification on a C18 cartridge gave 3.7 mg of pure E11.

### Example E12: 6-(3-aminocarbonylphenyl)-1-methyl-9H-pyrido[3,4-b]indole

The synthesis and purification of E12 was carried out applying the procedure described for E21 using 3-aminocarbonylphenylboronic acid. The reaction yielded 17.5 mg of E12.

### Example E13: 6-(3-acetylphenyl)-1-methyl-9H-pyrido[3,4-b]indole

The synthesis and purification of E13 was carried out applying the procedure described for example E21 using 3-acetylphenylboronic acid. The reaction yielded 82 mg of E13.

### Example E14: 6-(3-methoxycarbonylphenyl)-1-methyl-9H-pyrido[3,4-b]indole

The synthesis and purification of E14 was carried out applying the procedure described for example E21 using 3-methoxycarbonylphenylboronic acid. The reaction yielded 71 mg of E14.

### Example E15: 6-(3-(acetoxymethyl)phenyl)-1-methyl-9H-pyrido[3,4-b]indole

6-(3-hydroxymethylphenyl)-1-methyl-9H-pyrido[3,4-b]indole (example E18; 10.6 mg; 0.037 mmol) was dissolved in pyridine (0.1 mL) and acetic anhydride (0.1 ml). After one hour at 40°C, the reaction was stopped by adding methanol (0.5 mL), water (2 mL) and 1M NaOH (0.18 mL) to the reaction mixture. Purification on a C18 cartridge gave 10.6 mg of E15.

### Example E16: 6-(3,5-bis(acetoxymethyl)phenyl)-1-methyl-9H-pyrido[3,4-b]indole

6-(3,5-bis(hydroxymethyl)phenyl)-1-methyl-9H-pyrido[3,4-b]indole (example E20; 8 mg; 0.025 mmol) was dissolved in pyridine (0.1 mL) and acetic anhydride (0.1 ml). After one hour at 40°C, the reaction was stopped by adding water (5 mL) to the reaction mixture. Purification on a C18 cartridge gave 6.1 mg of pure E16.

### Example E17: 6-(3-hydroxyphenyl)-1-methyl-9H-pyrido[3,4-b]indole

The synthesis and purification of E17 was carried out applying the procedure described for example E21 using 3-hydroxyphenylboronic acid. The reaction yielded 37 mg of E17.

### Example E18: 6-(3-hydroxymethylphenyl)-1-methyl-9H-pyrido[3,4-b]indole

The synthesis and purification of E18 was carried out applying the procedure described for example E21 using 3-hydroxymethylphenylboronic acid. The reaction yielded 19.8 mg of E18.

### Example E19: 6-(3-(1-hydroxyethyl)phenyl)-1-methyl-9H-pyrido[3,4-b]indole

6-(3-acetylphenyl)-1-methyl-9H-pyrido[3,4-b]indole (example E13; 20 mg; 0.067 mmol) was dissolved in isopropanol/methanol (1 mL + 1 mL) and a solution of sodium borohydride (25 mg) in isopropanol (1 mL) was added dropwise until an LC-MS analysis showed completion of the reaction. After quenching the excess of borohydride with 1 M HCl and adjusting the solution to pH 7-8 with 1 M NaOH, purification on a C18 cartridge gave 15.8 mg of E19.

### Example E20: 6-(3,5-bis(hydroxymethyl)phenyl)-1-methyl-9H-pyrido[3,4-b]indole

6-(3,5-diformylphenyl)-1-methyl-9H-pyrido[3,4-b]indole was synthesized from 3,5-diformylphenylboronic acid and 6-bromo-1-methyl-9H-pyrido[3,4-b]indole (200 mg) in a procedure similar to the one described in example E21. Without further purification this product was reduced with sodium borohydride in methanol/isopropanol. Purification by preparative HPLC on a C18 column yielded 27.7 mg of pure E20.

### Example E21: 6-(3-cyanophenyl)-1-methyl-9H-pyrido[3,4-b]indole

6-bromo-1-methyl-9H-pyrido[3,4-b]indole (0.77 mmol, 200 mg), 3-cyanophenylboronic acid (339 mg, 2.31 mmol), potassium carbonate (858 mg, 6.16 mmol) and tetrakis(triphenylphosphine)palladium (37 mg, 0.032 mmol) were stirred in a mixture of dioxane (70 mL) and water (2 mL) for 29 hours at 105°C. After evaporating of the solvent, the reaction mixture was redissolved in a mixture of methanol, formic acid and water and purified by preparative HPLC on a C18-column eluting with a gradient of water and acetonitrile (with 0.1% formic acid). Pure fractions were combined and dried to give 84 mg of E21.

### Example E22: 6-(4-cyanophenyl)-1-methyl-9H-pyrido[3,4-b]indole

The synthesis and purification of E22 was carried out applying the procedure described for example E21 using 4-cyanophenylboronic acid. The reaction yielded 17 mg of E22.

### Example E23: 6-(3-cyanomethylphenyl)-1-methyl-9H-pyrido[3,4-b]indole

The synthesis and purification of E23 was carried out applying the procedure described for example E21 using 3-cyanomethylphenylboronic acid. The reaction yielded 71 mg of E23.

### Example E24: 6-(5-cyano-2-fluorophenyl)-1-methyl-9H-pyrido[3,4-b]indole

The synthesis and purification of E24 was carried out applying the procedure described for example E21 using 5-cyano-2-fluorophenylboronic acid. The reaction yielded 33 mg of E24.

### Example E25: 6-(pyridine-3-yl)-1-methyl-9H-pyrido[3,4-b]indole

Pyridin-3-ylboronic acid (0.459 mmol; 56 mg), 6-bromo-1-methyl-9H-pyrido[3,4-b]indole (0.383 mmol; 100 mg), K₂CO₃ (0.92 mmol; 127 mg) and tetrakis(triphenylphosphine)palladium (0.0046 mmol; 53 mg) in dioxane (50 mL) were heated at 85°C for 12 hours while stirring. The reaction mixture was adsorbed onto a cation exchanger cartridge (SCX), which was then washed with methanol and eluted with methanol/aq. ammonia (2%). Further purification by preparative HPLC gave 2 mg of E25.

### Example E26: 6-(1H-indol-5-yl)-1-methyl-9H-pyrido[3,4-b]indole

The synthesis and purification of E26 was carried out applying the procedure described for example E21 using 1H-indol-5-ylboronic acid. The reaction yielded 62 mg of E26.

### Example E27: 6-(((2H-tetrazol-5-yl)methyl)phenyl)-1-methyl-9H-pyrido[3,4-b]indole

To a solution of 6-(3-cyanomethylphenyl)-1-methyl-9H-pyrido[3,4-b]indole (example E23; 15 mg; 0.05 mmol) in iso-propanol (1 mL) a solution of sodium azide (75 mg; 1.15 mmol) and ZnCl₂ (40 mg; 0.29 mmol) in water (0.4 mL) was added. After heating at 95°C for four days the pure product, E27 (2.5 mg), was recovered from a precipitate inside the reflux cooler.

### Example E28: 6-(5-methylfuran-2-yl)-1-methyl-9H-pyrido[3,4-b]indole

E28 (59 mg) was prepared from 4,4,5,5-tetramethyl-2-(5-methylfuran-2-yl)-1,3,2-dioxaborolane using the procedure described in example E2.

### Example E29: 6-(2,3-dihydrobenzofuran-5-yl)-1-methyl-9H-pyrido[3,4-b]indole

E29 (7 mg) was prepared from 2,3-dihydrobenzofuran-5-ylboronic acid using the procedure described in example E7.

### Example E30: 6-(benzo[d][1,3]dioxol-5-yl)-1-methyl-9H-pyrido[3,4-b]indole

The synthesis and purification of E30 was carried out applying the procedure described for example E21 using benzo[d][1,3]dioxol-5-ylboronic acid. The reaction yielded 74 mg of E30.

### Example E31: 6-(5-formylfuran-2-yl)-1-methyl-9H-pyrido[3,4-b]indole

The synthesis and purification of E31 was carried out applying the procedure described for example E21 using 5-formylfuran-2-ylboronic acid. The reaction yielded 20 mg of E31.

### Example E32: 6-(5-hydroxymethylfuran-2-yl)-1-methyl-9H-pyrido[3,4-b]indole

A solution of 6-(5-formylfuran-2-yl)-1-methyl-9H-pyrido[3,4-b]indole (10 mg; example E31) in acetonitrile (1 mL) was titrated with a solution of sodium borohydride (14 mg) in acetonitrile (1 mL) until full conversion of the starting material was observed by LC-MS analysis. After quenching the excess of borohydride with 1M HCl and adjusting the solution to pH 7-8 with 1M NaOH, purification on a C18 cartridge gave 11.2 mg of E32.

### Example E33: 6-(3-aminophenyl)-1-methyl-9H-pyrido[3,4-b]indole

The synthesis of E33 was carried out applying the procedure described for E21 using 3-aminophenylboronic acid. After preparative HPLC with water/acetonitrile containing 0.1% formic acid the drying of the product containing fractions led to conversion of approximately 50% of the product to 6-(3-formamidophenyl)-1-methyl-9H-pyrido[3,4-b]indole (example E34). The pure compounds E33 (28.4 mg) and E34 (14.9 mg) were obtained after another preparative HPLC separation followed by removal of the formic acid using C18 cartridges.

### Example E34: 6-(3-formamidophenyl)-1-methyl-9H-pyrido[3,4-b]indole

See Example E33.

### Example E35: 6-(3-aminocarbonylphenyl)-1-ethyl-9H-pyrido[3,4-b]indole

6-bromo-1-ethyl-9H-pyrido[3,4-b]indole (Intermediate 12; 0.36 mmol, 108 mg), 3-carbamoylphenylboronic acid (185 mg, 1.09 mmol), potassium carbonate (254 mg, 1.82mmol) and tetrakis(triphenylphosphine)palladium (20 mg, 0.014 mmol) were stirred in a mixture of dioxane (20 mL) and water (0.5 mL) for two hours at reflux conditions under nitrogen atmosphere. After evaporating the solvent, the reaction mixture was redissolved in methanol and filtered through a C18-cartridge (1 g). The filtrate was purified further by preparative HPLC on a C18-column eluting with a gradient of water and acetonitrile (with 0.1% formic acid). Pure fractions were combined and dried to give 18.3 mg of E35.

### Example E36: 6-(2,3-dihydrobenzofuran-5-yl)-1-trifluoromethyl-9H-pyrido[3,4-b]indole

6-bromo-1-trifluoromethyl-9H-pyrido[3,4-b]indole (Intermediate I3; 0.25 mmol, 80 mg), 2,3-dihydrobenzofuran-5-ylboronic acid (104 mg, 0.48 mmol), potassium carbonate (176 mg, 0.80 mmol) and tetrakis(triphenylphosphine)palladium (12 mg, 0.006 mmol) were stirred in a mixture of dioxane (10 mL) and water (0.3 mL) for 17 hours at reflux conditions under nitrogen atmosphere. After evaporating the solvent, the reaction mixture was redissolved in methanol and filtered through a C 18-cartridge (1 g). The filtrate was purified further by preparative HPLC on a C18-column eluting with a gradient of water and acetonitrile (with 0.1% formic acid) followed by open column chromatography on silica gel (conditioned with 5 % (w/w) of concentrated aq. ammonia solution)eluting with dichloromethane/n-heptane (2:1). Pure fractions were combined and dried to give 48.0 mg of E36.

### Example E37: 6-(2,3-dihydrobenzofuran-5-yl)-1-ethyl-9H-pyrido[3,4-b]indole

6-bromo-1-ethyl-9H-pyrido[3,4-b]indole (Intermediate I2; 0.38 mmol, 105 mg), 2,3-dihydrobenzofuran-5-ylboronic acid (191 mg, 1.16 mmol), potassium carbonate (257 mg, 1.88 mmol) and tetrakis(triphenylphosphine)palladium (25 mg, 0.022 mmol) were stirred in a mixture of dioxane (25 mL) and water (1 mL) for two hours at reflux conditions under nitrogen atmosphere. After evaporating the solvent, the reaction mixture was redissolved in methanol and filtered through a C18-cartridge (1 g). The filtrate was purified further by preparative HPLC on a C 18-column eluting with a gradient of water and acetonitrile (with 0.1% formic acid). Pure fractions were combined and adjusted to pH>9 with ammonia. The resulting precipitate was filtered, washed with water and dried to give 25 mg of E37.

### Example E38: 6-(2,3-dihydrobenzofuran-5-yl)-1-propyl-9H-pyrido[3,4-b]indole

6-bromo-1-propyl-9H-pyrido[3,4-b]indole (Intermediate I4; 0.69 mmol, 200 mg), 2,3-dihydrobenzofuran-5-ylboronic acid (346 mg, 2.08 mmol), potassium carbonate (484 mg, 3.46 mmol) and tetrakis(triphenylphosphine)palladium (35 mg, 0.028 mmol) were stirred in a mixture of dioxane (40 mL) and water (1 mL) for two hours at reflux conditions under nitrogen atmosphere. After evaporating the solvent, the reaction mixture was redissolved in methanol and filtered through a C18-cartridge (1 g). The filtrate was purified further by preparative HPLC on a C18-column eluting with a gradient of water and acetonitrile (with 0.1% formic acid). Pure fractions were combined and dried to give 93.7 mg of E38.

### Example E39: 6-(2,3-dihydrobenzofuran-5-yl)-1-isopropyl-9H-pyrido[3,4-b]indole

6-bromo-1-isopropyl-9H-pyrido[3,4-b]indole (Intermediate I5; 0.17 mmol, 50 mg), 2,3-dihydrobenzofuran-5-ylboronic acid (62 mg, 0.345 mmol), potassium carbonate (120 mg, 0.87 mmol) and tetrakis(triphenylphosphine)palladium (22 mg, 0.019 mmol) were stirred in dioxane (10 mL) for one hours at reflux conditions under nitrogen atmosphere. After evaporating the solvent, the reaction mixture was redissolved in methanol and filtered through a C18-cartridge (1 g). The filtrate was purified further by preparative HPLC on a C 18-column eluting with a gradient of water and acetonitrile (with 0.1% formic acid). Pure fractions were combined and dried to give 10 mg of E39.

The compounds were tested for pharmacological activity. In each of the tests, each compound was tested at 10 µM in duplicates. Each test included the DNA synthesis inhibitor doxorubicin (IC₅₀ of 0.085 µM) and the apoptosis-inducing protein kinase inhibitor staurosporine (IC₅₀ of 0.031 µM), as well as a DMSO control. The percentage of response relative to the DMSO control was then calculated for the mean value of the duplicates of each test. The values of the three tests were finally averaged and the standard deviations calculated.

Jurkat cells (10 000 cells/well) were incubated in the presence or the absence of compounds, doxorubicin or staurosporine (10 µM). Twenty-two hours later, cells were incubated with resazurin (20 µg/ml) for 4 hours and fluorescence was measured at 590 nm. The results presented in Table 1 are the composite of three independent experiments run in duplicates.

Compounds were assessed for their ability to induce apoptosis of Jurkat cells at 2 and 10 micromolar after 4 and 22 hours. The results are shown in Table 2.

**Table 2**

| **Compound** | **Apoptosis-inducing effect on Jurkat cells: caspase 3 and 7 activity compared to solvent control (%)** | | | |
|---|---|---|---|---|
| | **10µM after 4h** | **10µM after 22h** | **2 µM after 4h** | **2 µM after 22h** |
| E3 | 12.7 | 743.7 | -13.6 | 689.0 |
| E25 | 18.7 | 405.7 | -0.6 | 624.4 |
| E6 | 11.2 | 585.1 | 9.2 | 482.6 |
| E7 | -10.2 | 548.1 | -7.6 | 616.1 |
| E1 | -10.0 | 535.9 | -8.5 | 618.5 |

In order to firmly establish that the compounds of the invention cause cytotoxicity through apoptosis, IC₅₀ of five compounds of the invention, doxorubicin and staurosporin were determined in the Alamar Blue and in the caspase assays; results are presented in Table 1. Importantly, the IC₅₀ determined in these two assays were largely overlapping. These observations indicated that the loss of viability observed in the Alamar Blue assay was likely due to apoptosis revealed by the activity of caspases 3 and 7.

## Claims

1. A compound of general formula (I): 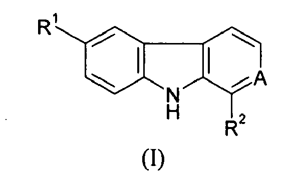 wherein
A is CH or N,
R¹ is aryl or heteroaryl selected from nitrogen containing compounds and oxygen containing compounds optionally substituted by up to four substituents independently selected from:
1. halo,
2. C₁-C₆ alkyl,
3. C₁-C₆ haloalkyl,
4. C₁-C₆ alkoxy,
5. C₁-C₆ haloalkoxy,
6. NHR³, (C₁-C₄ alkylene)NHR³;
7. NHC(O)R³, (C₁-C₄ alk-ylene)NHC(O)(C₁-C₆ alkyl),
8. C(O)NR³R⁴, (C₁-C₄ alkylene)C(O)N R³R⁴;
9. C(O)R³, (C₁-C₄ alkylene)C(O)R³;
10. C(O)O(C)-C₆ alkyl), (C₁-C₄ alkylene)C(O)O(C₁-C₆ alkyl),
11. OCCO)(C₁-C₆ alkyl), (C₁-C₄ alkylene)OC(O)(C₁-C₆ alkyl),
12. OH, (C₁-C₄ alkylene)OH,
13. CN, (C₁-C₄ alkylene)CN,
14. aryl or heteroaryl selected from nitrogen containing compounds and oxygen containing compounds optionally substituted;
wherein each R³ and each R⁴ is independently H or C₁-C₆ alkyl;
R² is C₁-C₆ alkyl optionally substituted with halo;
or a pharmaceutically acceptable salt or solvate thereof;
provided that
if R¹ is a phenyl group, it is not substituted by any of the substituents 1 to 12 or 14 at the 4-position,
if R¹ is a phenyl group, it is not substituted by any of the substituents 2 to 14 at the 2-position
if R¹ is an aryl, it is not a naphthalenyl group, anthracenyl or phenanthrenyl group,
if R¹ is an heteroaryl selected from nitrogen containing compounds, it is not a pyrimidinyl or a pyridazinyl group.

2. A compound according to claim 1 wherein R² is C₁-C₄ alkyl optionally substituted with halo.

3. A compound according to claim 1 or claim 2, wherein A is nitrogen (N).

4. A compound according to any one of claims 1 to 3, wherein R¹ is phenyl, pyridyl, benzopyridyl, pyrrolyl, tetrazolyl, indolyl, indolinyl, furanyl, benzofuranyl, dihydrobenzofuranyl, and benzodioxolyl, optionally substituted as described above, any of which is optionally substituted.

5. A compound according to claim 4, wherein R¹ a phenyl group which is unsubstituted, which has a substituent at the 3-position, which has two substituents at the 3- and the 5-position, or which is selected from 3-pyridyl, 2,3-benzofuran-5-yl, 5-substituted furan-2-yl, benzo[d][1,3]dioxo-5-yl and 1H-indol-5-yl, wherein, in substituted phenyl or furanyl groups, other substituents may also be present.

6. A compound according to any one of claims 1 to 5 wherein R¹ is unsubstituted or has 1 to 4 substituents selected from
- halo,
- C₁-C₄ alkyl,
- C₁-C₄ haloalkyl,
- C₁-C₄ alkoxy,
- C₁-C₄ haloalkoxy,
- NHR⁵, (C₁-C₂ alkylene)NHR⁵;
- NHC(O)R⁵, (C₁-C₂ alkylene)NHC(O)(C₁-C₄ alkyl),
- CCO)NR⁵R⁶, (C₁-C₂ alkylene)CCO)NR⁵R⁶;
- C(O)R⁵; (C₁-C₂ alkylene)C(O)R⁵;
- C(O)O(C₁-C₄ alkyl), (C₁-C₂ alkylene)C(O)O(C₁-C₄ alkyl),
- OC(O)(C₁-C₄ alkyl), (C₁-C₂ alkylene)OC(O)(C₁-C₄ alkyl),
- OH, (C₁-C₂ alkylene)OH,
- CN, (C₁-C₂ alkylene)CN, or
- aryl or heteroaryl selected from nitrogen containing compounds and oxygen containing compounds optionally substituted;
wherein each R⁵ and each R⁶ is independently H or C₁-C₄ alkyl.
Any of the suitable substituents for R¹ described above may be substituted with one or more chloro or fluoro substitutents.

7. A compound according to claim 1 selected from:
6-phenyl-1-methyl-9H-pyrido[3,4-b]indole;
6-(3-chlorophenyl)-1-methyl-9H-pyrido[3,4-b]indole;
6-(3,5-dichlorophenyl)-1-methyl-9H-pyrido[3,4-b]indole,
6-(2-fluorophenyl)-1-methyl-9H-pyrido[3,4-b]indole;
6-(3-fluorophenyl)-1-methyl-9H-pyrido[3,4-b]indole;
6-(3-(trifluoromethyl)phenyl)-1-methyl-9H-pyrido[3,4-b]indole;
6-(5-ethoxy-2-fluorophenyl)-1-methyl-9H-pyrido[3,4-b]indole;
6-(3,5-dimethylphenyl)-1-methyl-9H-pyrido[3,4-b]indole;
6-(3-methoxyphenyl)-1-methyl-9H-pyrido[3,4-b]indole;
6-(3-(aminomethyl)phenyl)-1-methyl-9H-pyrido[3,4-b]indole;
6-(3-(acetamidomethyl)phenyl)-1-methyl-9H-pyrido[3,4-b]indole;
6-(3-aminocarbonylphenyl)-1-methyl-9H-pyrido[3,4-b]indole;
6-(3-acetylphenyl)-1-methyl-9H-pyrido[3,4-b]indole;
6-(3-methoxycarbonylphenyl)-1-methyl-9H-pyrido[3,4-b]indole;
6-(3-(acetoxymethyl)phenyl)-1-methyl-9H-pyrido[3,4-b]indole;
6-(3,5-bis(acetoxymethyl)phenyl)-1-methyl-9H-pyrido[3,4-b]indole;
6-(3-hydroxyphenyl)-1-methyl-9H-pyrido[3,4-b]indole;
6-(3-hydroxymethylphenyl)-1-methyl-9H-pyrido[3,4-b]indole;
6-(3-(1-hydroxyethyl)phenyl)-1-methyl-9H-pyrido[3,4-b]indole;
6-(3,5-bis(hydroxymethyl)phenyl)-1-methyl-9H-pyrido[3,4-b]indole;
6-(3-cyanophenyl)-1-methyl-9H-pyrido[3,4-b]indole,
6-(4-cyanophenyl)-1-methyl-9H-pyrido[3,4-b]indole;
6-(3-cyanomethylphenyl)-1-methyl-9H-pyrido[3,4-b]indole;
6-(5-cyano-2-fluorophenyl)-1-methyl-9H-pyrido[3,4-b]indole;
6-(pyridine-3-yl)-1-methyl-9H-pyrido[3,4-b]indole;
6-(1H-indol-5-yl)-1-methyl-9H-pyrido[3,4-b]indole;
6-(((2H-tetrazol-5-yl)methyl)phenyl)-1-methyl-9H-pyrido[3,4-b]indole;
6-(5-methylfuran-2-yl)-1-methyl-9H-pyrido[3,4-b]indole;
6-(2,3-dihydrobenzofuran-5-yl)-1-methyl-9H-pyrido[3,4-b]indole;
6-(benzo[d][1,3]dioxol-5-yl)-1-methyl-9H-pyrido[3,4-b]indole;
6-(5-formylfuran-2-yl)-1-methyl-9H-pyrido[3,4-b]indole;
6-(5-hydroxymethylfuran-2-yl)-1-methyl-9H-pyrido[3,4-b]indole;
6-(3-aminophenyl)-1-methyl-9H-pyrido[3,4-b]indole;
6-(3-formamidophenyl)-1-methyl-9H-pyrido[3,4-b]indole;
6-(3-aminocarbonylphenyl)-1-ethyl-9H-pyrido[3,4-b]indole;
6-(2,3-dihydrobenzofuran-5-yl)-1-trifluoromethyl-9H-pyrido[3,4-b]indole;
6-(2,3-dihydrobenzofuran-5-yl)-1-ethyl-9H-pyrido[3,4-b]indole;
6-(2,3-dihydrobenzofuran-5-yl)-1-propyl-9H-pyrido[3,4-b]indole;
6-(2,3-dihydrobenzofuran-5-yl)-1-isopropyl-9H-pyrido[3,4-b]indole
and pharmaceutically acceptable salts or solvates thereof.

8. A compound according to any one of claims 1 to 7 for use in medicine.

9. A compound according to any one of claims 1 to 7 for use as an intermediate in the synthesis of a medicament..

10. The use of a compound according to any one of claims 1 to 7 in the preparation of an agent for the treatment or prevention of a proliferative disorder.

11. A method for inhibiting tubulin polymerization by administering to a patient a therapeutically effective amount of a compound according to any one of claims I to 7.

12. A method for the treatment or prevention of a proliferative disorder, the method providing administering to a patient in need of such treatment a therapeutically effective amount of a compound according to any one of claims 1 to 7.

13. A pharmaceutical or veterinary composition comprising a compound according to any one of claims 1 to 7 together with a pharmaceutically or veterinarily acceptable carrier.

14. A product comprising a compound according to any one of claims 1 to 7 and one or more of additional therapeutic agents as a combined preparation for simultaneous, separate or sequential use in the treatment of a proliferative disorder.

15. A process for the preparation of a compound according to any one of claims 1 to 7, the process comprising reacting a compound of the formula: 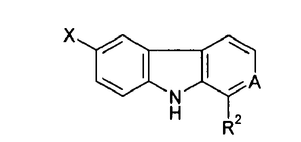 wherein A and R² are as defined for general formula (I) and X is a leaving group; with a compound of the formula:
R¹-B(OH)₂
where R¹ is as defined for general formula (I).
